# EUROPEAN PATENT APPLICATION

(11) **EP 2 554 112 A1**
(43) Date of publication of application: **06.02.2013**
(21) Application number: 11759606.4
(22) Date of filing: 25.03.2011
(51) Int. Cl.: A61B 5/0476, A61B 5/0408, A61B 5/0478, A61B 5/0492, A61F 7/12

(54) **LOCALIZED CEREBRUM COOLING PROBE AND BRAIN-FUNCTION MAPPING DEVICE**

(30) Priority: 26.03.2010 JP 2010071321
(71) Applicant: Yamaguchi University, Yamaguchi 753-8511 (JP)
(72) Inventor: FUJII, Masami, Ube-shi Yamaguchi 755-8505 (JP); FUJIOKA, Hiroshi, Ube-shi Yamaguchi 755-8505 (JP); SUZUKI, Michiyasu, Ube-shi Yamaguchi 755-8505 (JP); SAITO, Takashi, Ube-shi Yamaguchi 755-8611 (JP); KAJIWARA, Koji, Ube-shi Yamaguchi 755-8505 (JP); YOSHIKAWA, Koichi, Ube-shi Yamaguchi 755-8505 (JP); NOMURA, Sadahiro, Ube-shi Yamaguchi 755-8505 (JP)
(74) Representative: TER MEER - STEINMEISTER & PARTNER GbR
(86) International application number: PCT/JP2011/057424
(87) International publication number: WO 2011/118802

(57) **Abstract**

A cerebral local portion cooling probe is composed so that a handheld module includes a Peltier device and a coolant liquid circulating part arranged so as to contact with the Peltier device and wirings, conduits and a heat radiating part are connected to this handheld module. Circulation of coolant liquid and operation of the Peltier device are implemented by means of a control unit, then the tip end of the handheld module is caused to contact with a cerebral local portion to be cooled and a state of the cerebral local portion is monitored in accordance with responses to the cooling. A cerebral function mapping device comprises a mapping module in which a plurality of cooling modules are arranged, each cooling module including a Peltier device and a coolant liquid circulating part in a manner similar to that of the cerebral local portion cooling probe. Wirings, conduits and a heat radiating part are connected to respective cooling modules. One of the cooling modules is selectively operated by a control unit. The mapping device includes means for inputting and accumulating data on responses when the cerebral local portion is cooled and for creating mapping data. Thereby, safety and operability can be improved in monitoring a state of the cerebral local portion and in mapping cerebral function.

## Description

### FIELD OF THE INVENTION

The present invention relates to a cerebral local portion cooling probe and a cerebral mapping device using the probe.

### BACKGROUND OF THE INVENTION

When performing a neurosurgical treatment for patients with diseases such as brain disease, and intractable epilepsy, it is quite important to evaluate (or implement mapping of) local cerebral functions in individual patients in detail to avoid surgical complications.

At present, in order to detect limb movement function early, a technique is used in which an extent of resection is determined after a cerebral function (language area) mapping is performed with motor evoked potential (MEP) or surgery in a state where a patient is awakened.

Conventional cerebral function mapping is disclosed in Leading Edge Functional Neurosurgery (Chapter 9, "Brain and Spinal Cord Function Mapping") published by Research Center for Medical Innovation and Technology on June 20, 2002, pp. 329-339 (Non-Patent Documents 1) and in Nerve Monitoring Atlas for Neurosurgery, (Chapter 2, "Tumor Resection in Broca Language Area") published by Igaku-Shoin on February 1, 2003, pp.29-36 (Non-Patent Document 2). In the conventional cerebral function mapping, cerebral local regions are electrically stimulated to perform cerebral function mapping in accordance with reactions in response to the electrical stimulation. In such a cerebral function mapping, there are some problems such as induction of convulsion fit due to the electrical stimulation and dispersion in analysis of measured results due to a setting of intensity of the electrical stimulation.

Also, it is disclosed in Sartorius CJ, Berger MS: Rapid termination of intraoperative stimulation-evoked seizures with application of cold Ringer's lactate to the cortex. (J Neurosurg 88:349-351; 1998) (Non-Patent Document 3) that the convulsion fit is restricted by pouring cool water of 4°C onto a brain surface in cerebral function mapping in an operation, when convulsion fit is induced by the electrical stimulation.

Further, devices for restricting convulsion fit by cooling the cerebral local regions are disclosed in Japanese Patent Application Laid-Open No.2006-141993 (Patent Document 1) and Japanese Patent Application Laid-Open No.2007-209523 (Patent Document 2). Also, devices that the inventor proposed are disclosed in Japanese Patent No.3843054 (Patent Document 3) and Japanese Patent Application No.2009-236290 (Patent Document 4).

### SUMMARY OF THE INVENTION

Conventional cerebral function mapping was to evaluate and monitor cerebral function at local portions using electric stimulation, which induced convulsion fit due to the electric stimulation or generated dispersion in analysis of a measurement result due to setting of intensity of the electric stimulation. Actually, the electric stimulation often caused the convulsion fit in mapping of language area in a surgery where a patient is awakened. Accordingly, this was one of risks in the surgery where the patient is awakened and a problem highly necessary to be solved thereafter.

The present invention is to achieve the above described objects. The cerebral local portion cooling probe according to the present invention comprises: a handheld module having a tubular casing and cooling means and temperature detecting means which are provided in the tubular casing at a tip end thereof; and a cooling control unit controlling temperature of a local portion of a cerebral surface to a predetermined temperature in accordance with temperature detection signal from the temperature detecting means, with the tip end of the handheld module being caused to contact with a cerebral local portion in order to cool the cerebral surface to the predetermined temperature thereby monitoring a state of the cerebral local portion, wherein the cooling means provided in the tubular casing at the tip end thereof is a Peltier device, a coolant liquid circulating part is provided so as to contact with the heat generating surface on the rear side of the Peltier device, and the coolant liquid circulating part and a heat radiating part provided externally of the handheld module are coupled by conduits to circulate coolant liquid and radiate heat.

A metal plate may be provided at the front surface of the Peltier device and exposed as a tip end surface of the tubular casing and a thermocouple and a brain wave detecting electrode may be embedded in the metal plate as the temperature detecting means. In this case, the metal plate may be provided at the front surface of the Peltier device and exposed as the tip end surface of the tubular casing, and the thermocouple may be embedded in the metal plate as the temperature detecting means, and the metal plate may also be used as the brain wave detecting electrode.

Further, a cerebral function mapping device according to the present invention comprises: a mapping module accommodating a plurality of cooling modules in a casing so that cooling tip end surfaces of the cooling modules are arranged in a same plane, each of these cooling modules including a tubular casing, cooling means and temperature detecting means arranged in the tubular casing at the tip end thereof; a cooling control unit for controlling a temperature of a local portion of a brain surface at a predetermined temperature according to temperature detection signals from the temperature detecting means in selected one of the cooling modules of the mapping module; cooling module selecting means for selecting one of the plurality of cooling modules in the mapping module and causing only the selected one of the cooling modules to perform cooling operation; response inputting means for inputting information on responses of the cerebral local portion monitored in accordance with the cooling operation of the selected cooling module; and mapping data creating means for accumulating the information input by the response inputting means and creating mapping data.

The cooling means of the respective cooling modules in the mapping module are Peltier devices. A coolant liquid circulating part may be arranged to contact with the heat generating surface at the rear side of the Peltier device. An inflow conduit and an outflow conduit of coolant liquid connected to the coolant liquid circulating part in the plurality of cooling modules may be connected to any one of a cold liquid heat radiating part and a warm liquid heat radiating part. Cold liquid of the coolant liquid is circulated through the selected one of the cooling modules and warm liquid of the coolant liquid may be circulated through the other cooling modules so as to radiate heat at the cold liquid heat radiating part and the warm liquid heat radiating part respectively.

A metal plate may be provided at a front surface of the Peltier device of each of the cooling modules in the mapping module and exposed as a tip end surface of the tubular casing of each of the cooling modules. A thermocouple may be embedded in the metal plate as the temperature detecting means. Also, the metal plate at the tip end surface of each of the cooling modules of the mapping module can be used as a brain wave detecting electrode.

The cerebral local portion cooling probe and the cerebral function mapping device using the probe according to the present invention use means for cooling a cerebral local portion to monitor a state of the cerebral local portion without inducing convulsion fit as in a case where a cerebral function is evaluated using conventional electric stimulation. Accordingly, safety in monitoring the state of the cerebral local portion can be improved. Also, in mapping the cerebral function, safety and operability can be improved.

### BRIEF DESCRIPTION OF THE DRAWINGS

FIG. 1 is a view showing a state where a brain is inspected using a cerebral local portion cooling probe according to the present invention.
FIG. 2 is a perspective view showing an appearance of a handheld module M.
FIG. 3 (a) is a cross-sectional view showing inside of the handheld module M taken along a longitudinal plane and FIG. 3 (b) is a perspective view showing individual elements contained in the casing on the distal end side.
FIG. 4 is a view showing a composition of the parts of a heat radiating part and a control processing unit in the cerebral local portion cooling probe.
FIG. 5 is a view showing a composition of a cerebral function mapping device according to the present invention.
FIG. 6 is a view showing a wiring form and a piping form connected to each of the cooling modules in the mapping module.
FIG. 7 is a view showing an example of a configuration of a flow path switch in the conduit for circulating the coolant liquid.
FIG. 8 is a view showing an appearance of a portion of the mapping module.
FIG. 9 is a view showing an example of items to be set and input and an arrangement of keys in a setting and inputting board.
FIG. 10 is a view showing an example of a display of cerebral function mapping data.

### DETAILED DESCRIPTION OF THE INVENTION

A cerebral local portion cooling probe and a cerebral function mapping device according to the present invention will be described below. The cerebral local portion cooling probe monitors a brain by cooling cerebral local portions using a cooling handheld module and examining responses of the cerebral local portions to the cooling. The cerebral function mapping device includes and uses a mapping module, in which a plurality of cooling modules used for the cerebral local portion cooling probe are arranged integrally, to perform cerebral function mapping.

### [Cerebral Local Portion Cooling Probe]

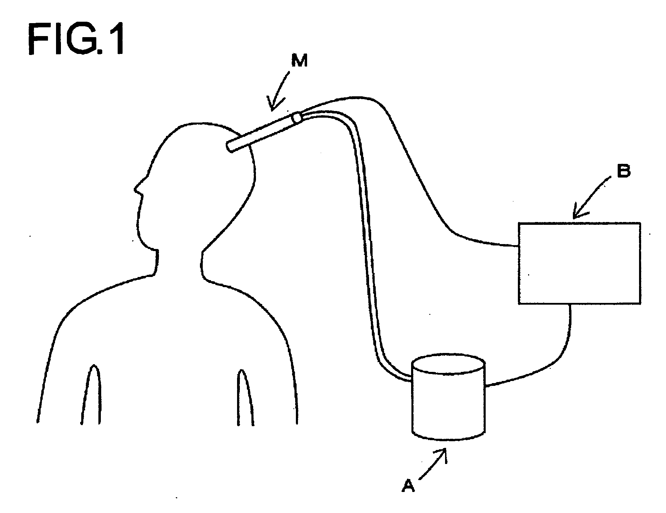

FIG. 1 shows a state in which a brain is monitored by using a cerebral local portion cooling probe according to the present invention. In FIG. 1, the cerebral local portion cooling probe includes a handheld module M, a heat radiating part A and a control processing unit B. The handheld module M has a tubular outer shape and includes a cooling part using a Peltier device and a detecting part with a sensor, having a function to cool a local portion of a brain through making its tip contact with the local portion.

The heat radiating part A includes a reservoir and a circulating pump for coolant liquid circulation so as to radiate heat generated by the Peltier device in the handheld module M. The heat radiating part A is connected to the handheld module M via an inflow conduit and an outflow conduit. The control processing unit B is connected to the handheld module M and to the heat radiating part A via wirings respectively to control operation of the Peltier device in the handheld module M and to control processing of signals, receiving temperature signals detected by a temperature detecting element, brain wave signals or the like obtained in the handheld module M, thus controlling operation of the coolant liquid circulating pump in the heat radiating part A. As for the coolant liquid, physiological saline water, Ringer's solution, pure water or the like with relatively high specific heat and high biological safety are used.

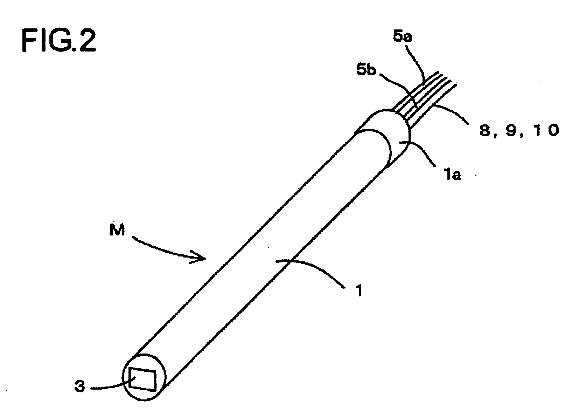

FIG. 2 is a perspective view showing an appearance of the handheld module M. The handheld module M is configured to make its tip end surface for cooling the cerebral local portions contact with the cerebral local portions. The handheld module M has a casing 1 formed of polypropylene in an aspect of biocompatibility with a smooth cylindrical outer shape to contain elements such as a Peltier device for cooling and a coolant liquid circulating part for radiating heat therein. A metal plate 3 formed of a material such as silver with high heat conductivity is disposed on the front surface of the cooling Peltier device to be exposed at the tip end surface of the casing 1. A rear end wall part 1a of the casing 1 is provided for closing the casing 1. An inflow conduit and an outflow conduit 5a, 5b formed of a flexible resin material for circulating the coolant liquid for radiating heat of the Peltier device, wirings 8, 9 for supplying detection signals from a temperature detecting element and a brain wave detecting electrode and a wiring 10 for operation of the Peltier device pass through the rear end wall part 1a. In FIG. 2, a single wiring is shown representing three of the wirings 8, 9 and 10.

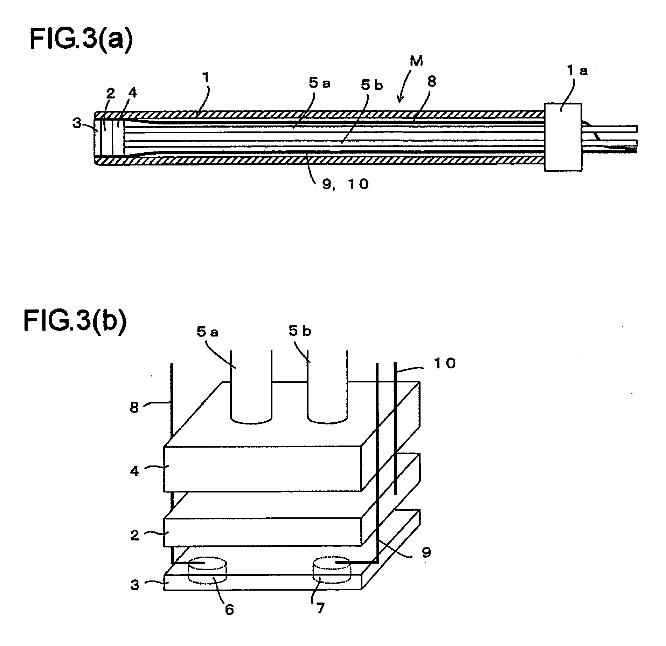

FIG. 3(a) shows the inside of the casing 1 of the handheld module M in a cross-sectional view taken along a longitudinal plane and FIG. 3 (b) is a perspective view showing individual elements contained in the casing on the distal end side. In FIGS. 3 (a) and 3(b), a metal plate 3 is provided on the front surface (cooling side) of the Peltier device 2 and a coolant liquid circulating part 4 is provided on the rear surface (heat generating side) of the Peltier device 2 is provided so that the front surface of the circulating part 4 contacts with the rear surface of the Peltier device 2. The metal plate 3 is formed of a material such as silver that is preferable for biocompatibility with high thermal conductivity. The inside of the coolant liquid circulating part 4 is box-shaped closed space. In order that the wall portion of the coolant liquid circulating part 4 transfers heat from the heat generating side of the Peltier device, the wall portion is formed of a material such as silver with high thermal conductivity, which does not cause kink when the probe is operated. The coolant liquid circulates through the coolant liquid circulating part 4 via the inflow conduit 5a and the outflow conduit 5b.

In FIG. 3(b), the metal plate 3, the Peltier device 2 and the coolant liquid circulating part 4 are shown separated. In the metal plate 3, the temperature detecting element 6 such as a thermocouple and the brain wave detecting electrode 7 are embedded. The wiring 8 transfers detection signals from the temperature detecting element 6. The wiring 9 transfers detection signals from the brain wave detecting electrode (ECoG electrode). The wiring 10 is provided for driving and controlling the Peltier device. The metal plate 3 itself can be used as the brain wave detecting electrode 7. In FIG. 3 (a), a single wiring is shown representing wirings 9 and 10.

As shown in FIG. 3 (a), the metal plate 3, the Peltier device 2 and the coolant liquid circulating part 4 are provided so that they closely contact with each other at the tip end of the casing 1 of the handheld module M, in which the metal plate 3 is exposed at the tip end surface of the casing 1, and surrounded and sealed by the wall portion of the casing 1. The tip end of the handheld module M contacts with the cerebral local portion. Accordingly, it is preferable that the metal portion 3 and the portion of the casing 1 surrounding and sealing the metal plate 3 are smoothly connected to each other and corners of the tip end are appropriately round-shaped.

The rear end wall part 1a closes the rear end part of the casing 1. The inflow conduit 5a the outflow conduit 5b and the wirings 8, 9 and 10 pass through the rear end wall part 1a. It is preferable that the size of the handheld module M with the cylindrical casing 1 and the rear end wall part 1a coupled together is of a length of 150 mm and an outer diameter 5 to 10 mm approximately. The inflow conduit 5a, the outflow conduit 5b and the wirings 8, 9, and 10 may be configured so that they can be attached to or detached from each other at the rear end wall part 1a or externally from the casing.

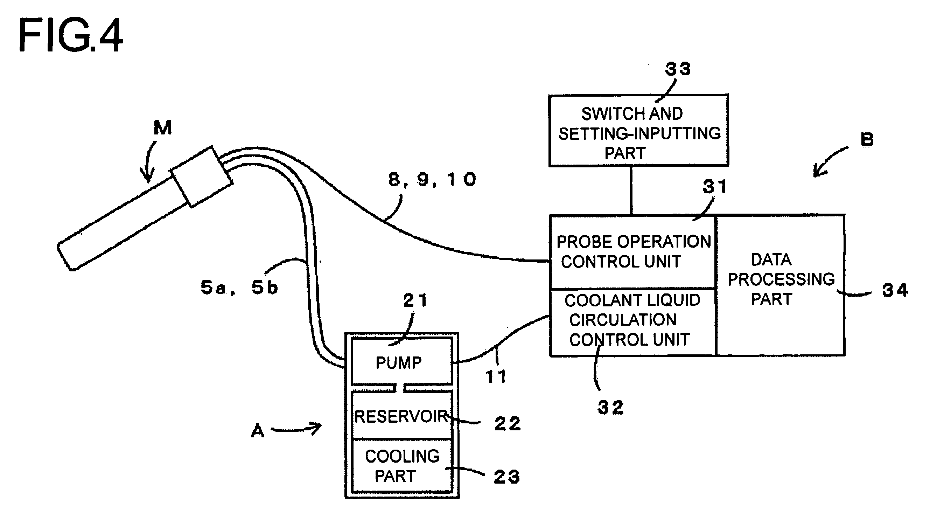

FIG. 4 is a view showing the composition of the heat radiating part A and the control processing unit B in more detail. The heat radiating part A includes a coolant liquid circulating pump 21, a reservoir 22 for accumulating coolant liquid and a reservoir cooling part 23. The coolant liquid circulating pump 21 is connected to the coolant liquid circulating part 4 in the handheld module M via the inflow conduit 5a and the outflow conduit 5b to circulate the coolant liquid between the coolant liquid circulating part 4 and the reservoir 22. The reservoir 22 is provided with the reservoir cooling part 23. Due to generated heat at the Peltier device, the temperature of the coolant liquid flown back to the reservoir is raised. The temperature of the coolant liquid is cooled to a predetermined temperature in the reservoir 22. The reservoir cooling part 23 includes cooling means by gas cooling or Peltier device cooling method to efficiently cool the coolant liquid in the reservoir 22. In order to cool the coolant liquid by the reservoir cooling part 23 efficiently, the cooling means may be a type configured so that the reservoir 22 is formed by a circulating conduit with a certain length to be cooled along and from around of the reservoir 22 by the cooling means.

The control processing unit B includes a probe operation control unit 31, a coolant liquid circulation control unit 32, a switch and setting-inputting part 33 and a data processing unit 34. The probe operation control unit 31 is connected to the handheld module M via the wirings 8, 9, and 10 to receive the signals from the temperature detecting element 6 via the wiring 8 and the signals from the brain wave detecting electrode 7 via the wiring 9. The probe operation control unit 31 controls the operation of the Peltier device 2 via the wiring 10 to cool the cerebral local portion to a predetermined temperature on the basis of data obtained by performing A/D conversion on the detected temperature signals and transmits data related to temperature control to the coolant liquid circulation control unit 32, which data are used for controlling circulation of the coolant liquid to radiate heat generated by cooling operation of the Peltier device 2 through the coolant liquid circulating in the coolant liquid circulating part 4. Also, data on the basis of the signals from the brain wave detecting electrode 7 are transmitted to the data processing unit 34. The data processing unit 34 includes a control circuit for performing A/D conversion on the detected signals, controlling temperature and controlling operation of the Peltier device and the like. In FIG. 4, a single wiring is shown representing the wirings 8, 9, and 10 and a single conduit is shown representing the inflow conduit 5a and the outflow conduit 5b.

The coolant liquid circulation control unit 32 includes a control circuit for controlling operation of the coolant liquid circulating pump to circulate the coolant liquid in accordance with data related to temperature control transmitted from the probe control unit 31. The switch and setting-inputting part 33 includes keys for switching on/off of the operation of the whole cerebral local portion cooling probe and setting and inputting cooling temperature.

The cerebral local portion is efficiently cooled by radiating the heat generated due to the operation of the Peltier device 2 with the coolant liquid circulating through the coolant liquid circulating part 4. Even if power of the Peltier device 2 is turned off, the circulation of the coolant liquid is not simultaneously stopped, namely it is necessary to continue the circulation for about 3 to 5 minutes. A reason for this is to inhibit a rapid rise of temperature of the probe caused by stopping the circulation of the coolant liquid simultaneously.

In FIG. 4, a power part for operating the cerebral local portion cooling probe, that is not shown in particular, supplies power to operate the pump 21, the reservoir cooling part 23 in the heat radiating part A and the respective control circuits of the control processing unit B. Such a type of power part commutating usual AC power by an adapter is used. Further, when using the cerebral local portion cooling probe and making the tip end of the handheld module contact with the cerebral local portion to be monitored, it is desirable to obtain positional information of the cerebral local portion. This is performed by, for example, using an existing navigation system.

The cerebral local portion cooling probe according to the present invention composed as described above drives the Peltier device to cool the cerebral local portion in a state where the coolant liquid circulates through the coolant liquid circulating part 4 in the handheld module so as to obtain a response to stimulation by cooling the cerebral local portion and monitor the state of the cerebral local portion.

The cerebral local portion cooling probe according to an embodiment of the present invention is used in a manner described as follows.
(a) Craniotomy is performed in a range necessary to monitor and excise lesion under general anesthesia.
(b) After completing the craniotomy, the anesthesia is discontinued to obtain an awakened state of a patient.
(c) Cerebral function is monitored with a focus on a language area or a motor area anatomically expected through making the tip end of the handheld module contact with the cerebral local portion and detecting brain wave signals with the tip end of the cerebral local portion cooling probe set at a predetermined temperature in an operating state of the probe.
   In cooling, the temperature of the tip end of the handheld module is set at a predetermined temperature in a range of 5 to 15°C and changes in neurological symptoms (a language function and a motor function of limbs) of the patient are observed before, during and after the cooling.
(d) The language function is evaluated according to naming of objects. The motor function is evaluated according to elevation of an upper limb and a lower limb, enclasping and unclasping of a hand and toe movement.
(e) Brain waves of cooled sites and around the cooled sites are measured and changes in electrical activity are also observed.
All of these measurements are completed in 60 minutes.
It is preferable to evaluate and set an optimum temperature and cooling period for effectively and efficiently performing these measurements using the cerebral local portion cooling probe.

### [Cerebral Function Mapping Device]

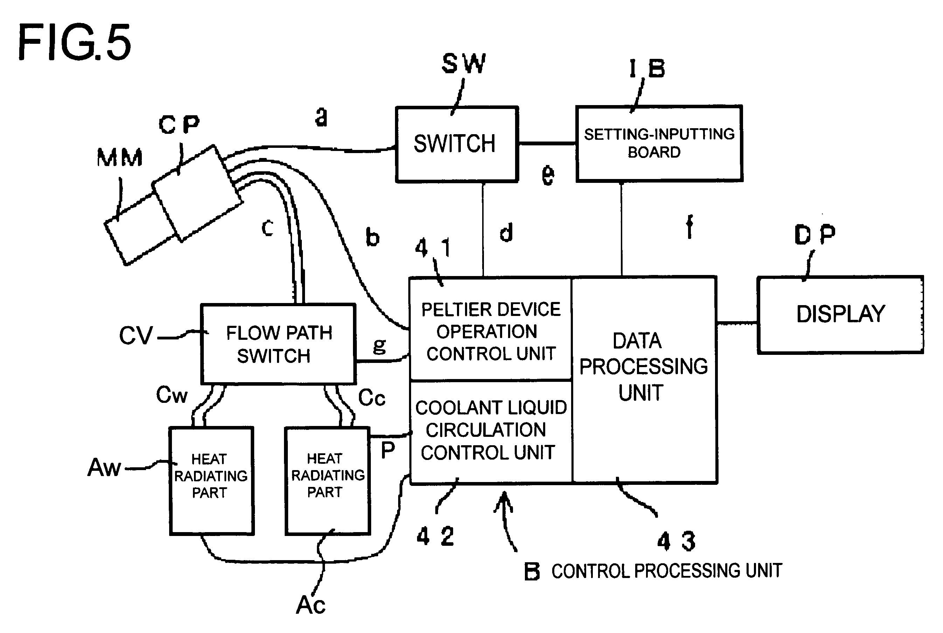

A cerebral function mapping device uses a plurality of cooling modules similar to the handheld module used for cooling the local portion with the cerebral local portion cooling probe, in which a mapping module is composed so that the cooling tip end surfaces of the plurality of cooling modules are disposed in a matrix in a plane. FIG. 5 shows a whole structure of the cerebral function mapping device including the mapping module composed as described above.

In FIG. 5, a mapping module MM is formed by arranging tip end surfaces of a plurality of cooling modules in a same plane. The cooling modules forming the mapping module are configured to operate selectively one of the Peltier devices disposed at the tip end of the tubular casing in a state where coolant liquid circulates through the coolant liquid circulating part contacting with the heat generating surface of the Peltier device in a manner similar as in case of the handheld module in the cerebral local portion cooling probe as shown in FIGS. 2 and 3. In mapping, a certain one of the plurality of cooling modules of the mapping module is selected to cool the local portion.

The composition for operating each mapping module MM includes a heat radiating part A and a control processing unit B basically in a manner similar to that of the cerebral local portion cooling probe as shown in FIG. 4. It differs particularly from the composition shown in FIG. 4 in that the heat radiating part A is separated into a warm liquid heat radiating part A_{W} and a cold liquid heat radiating part A_{C} so that warm liquid and cold liquid are switched by a flow path switch CV to be circulated and in that the composition includes a Peltier device switch SW for selectively operating the Peltier device in one of the plurality of cooling modules and a setting-inputting board IB for selecting one to be used from the plurality of cooling modules, specifying inspection positions and inspection items of the cerebral local portion by the mapping module MM and inputting responses. Each of the warm liquid heat radiating part A_{W} and the cold liquid heat radiating part A_{C} is provided with a coolant liquid circulating pump, a reservoir for accumulating the coolant liquid and a reservoir cooling part.

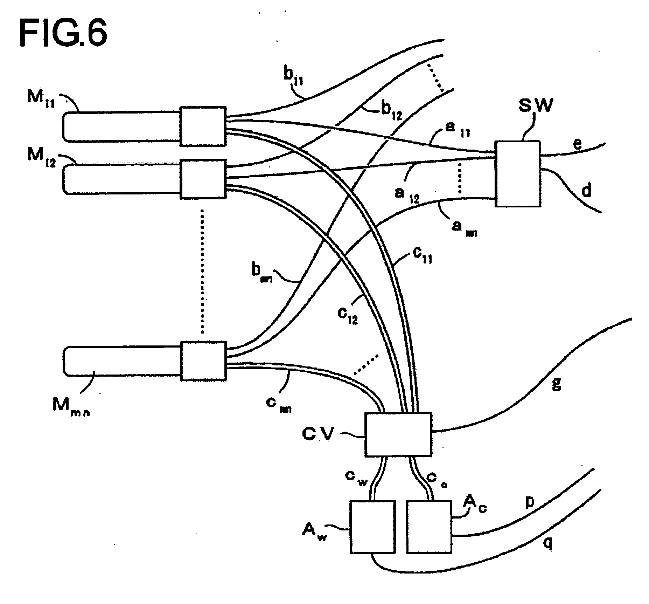

FIG. 6 shows a wiring form and a piping form connected to each cooling module of the mapping module MM. The cooling modules are shown arranged in a matrix of m x n represented by M₁₁, M₁₂, ..., Mₘₙ. Wirings a₁₁, a₁₂, ..., aₘₙ for operating the Peltier devices in the respective cooling modules connect the respective cooling modules M₁₁, M₁₂, ..., Mₘₙ to the Peltier device switch SW so that the wiring selected by switching the switch SW supplies the Peltier device of each of the cooling modules with operating current.

Wirings, representatively denoted by b₁₁, b₁₂, ..., bₘₙ, from the temperature detecting element and the brain wave detecting electrode of each of the cooling modules M₁₁, M₁₂, ... , Mₘₙ are respectively connected to a Peltier device operation control unit 41. Conduits (inflow conduits and outflow conduits), representatively denoted by c₁₁, c₁₂, ..., cₘₙ, from the coolant circulating part of each of the cooling modules M₁₁, M₁₂, ..., Mₘₙ are coupled with the flow path switch CV.

Flow paths are switched by the flow path switch CV so that the coolant liquid from the cool water heat radiating part A_{c} is circulated through the cooling module selected by switching the switch SW and the coolant liquid from the warm liquid heat radiating part A_{w} is circulated through the other cooling modules. The flow path switch CV is coupled with the warm liquid heat radiating part A_{w} by a warm liquid conduit c_{W}, and is also coupled with the cold liquid heat radiating part A_{c} by a cold liquid conduit c_{c}.

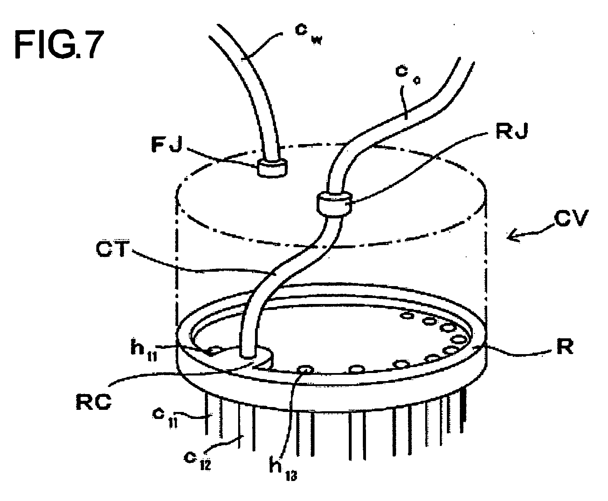

The flow path switch CV is configured as shown in FIG. 7, for example, so that the coolant liquid from the cool water heat radiating part A_{c} is circulated through the specific selected cooling module and the coolant liquid from the warm liquid heat radiating part A_{w} is circulated through the other cooling modules. In FIG. 7, positional up-down relationship of the conduits is inversed with respect to that shown in FIG. 6. The conduit c_{W} from the warm liquid heat radiating part A_{W} is coupled by a joint FJ with the top surface of a body part (shown by a dashed-line) of the flow path switch CV forming a cylindrical container and the conduit c_{c} from the cold liquid heat radiating part A_{c} is coupled with the top surface at the central position of the top surface of the body part of the flow path switch CV by a joint RJ.

The joint FJ is of a fixed type, through which warm liquid flows in and out of the body part of the flow path switch CV. The joint RJ, as a rotary joint, is coupled with an end of a selecting and coupling conduit CT. The other end of the selecting and coupling conduit CT is coupled with a joint RC protruding internally at a position of a rotatable ring-shaped actuator R arranged at a lower side of the body part of the flow path switch CV, where the joint RC is formed so as to make cold liquid flow downward further. It is preferable that the selecting and coupling conduit CT is formed by a material with low heat conductivity.

A plurality of through holes h₁₁, h₁₂, ... are formed at the lower end of the selecting and coupling conduit CT and on the same radial circumference. The inflow and outflow conduits c₁₁, c₁₂, ... coupled with the cooling modules are coupled to lower ends of the through holes respectively. The ring-shaped actuator R is driven by a pulse motor or a ultrasonic motor and the conduit (inflow and outflow conduits c₁₁, c₁₂, ...) selected by a movement of the joint in a circumferential direction is coupled with the conduit c_{c} through the selecting and coupling conduit CT. The other conduits that are not selected are coupled with the warm liquid conduit c_{w} through an inside of the body part of the flow path switch CV.

When the selecting and coupling conduit CT coupled with the joint RC is brought into a position of a desired one of the through holes (any of the through holes h₁₁, h₁₂, ...) by rotation of the ring-shaped actuator R, the cold liquid from the cold liquid heat radiating part A_{c} is circulated through only the selected conduit (inflow and outflow conduits) and the warm liquid from the warm liquid heat radiating part A_{w} is circulated through the other conduits.

With the warm liquid heat radiating part A_{w}, coolant liquid at below or over 35°C is circulated so that warm liquid at a temperature the same as that of the usual brain surface is normally circulated. With the cold liquid heat radiating part A_{c}, it is preferable that coolant liquid at a temperature of 5 to 15°C is circulated to cool the cerebral local portion by the selected cooling module. Also, while the conduits c₁₁, c₁₂, ... are representatively shown, a pair of conduits, i.e., an inflow conduit and an outflow conduit are provided for each cooling module so that the flow path switches CV corresponds to the pair of similar pair of conduits.

The body part of the flow path switch CV is for switching the circulations of the cold liquid and the warm liquid and not for reserve the liquid. Accordingly, the body part with a volume as little as possible is preferred. Also, the flow path switch CV operates to circulate the cold liquid only in the selected cooling module and the warm liquid in the other cooling modules. In an embodiment different from that described above, the cold liquid and the warm liquid can be switched and circulated by a changeover valve for each of the inflow conduits and the outflow conduits.

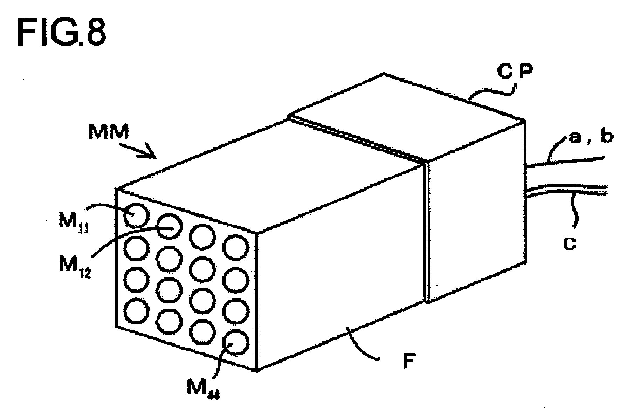

FIG. 8 shows an appearance of a portion of the mapping module in a case where 16 (4 x 4) cooling modules M₁₁, M₁₂, ..., M₄₄ are arranged in a matrix in a plane. Cooling tip end surfaces of the respective cooling modules are arranged in a same plane and the whole of the modules are held in a casing F. A rear side of a coupling part CP is a connecting parts for conduits and wirings to the heat radiating parts A_{w}, A_{c} and a Peltier device operation control unit 41 of the control processing unit B.

The control processing unit B includes the Peltier device operation control unit 41 for each of the cooling modules of the mapping module MM, the coolant liquid circulation control unit 42 and the data processing unit 43. The Peltier device operation control unit 41 has wirings b₁₁, b₁₂, ..., bₘₙ connected thereto from the temperature detecting element and the brain wave detecting electrode of each of the cooling modules, is connected to the Peltier device switch SW via a wiring d and to the flow path switch CV via a wiring g. The wiring d represents a wiring for receiving information related to the Peltier device selected by the switch SW and a wiring for supplying current for operation to the selected Peltier device.

The Peltier device operation control unit 41 receives the signals from each of the temperature detecting elements and the brain wave detecting electrodes via a wiring b (wirings b₁₁, b₁₂, ..., bₘₙ) from each of the cooling modules and supplies current for operating the selected Peltier device to the selected Peltier device so that the temperature of the cooled cerebral local portion related to the cooling module selected by the switch SW is a set value. Also, the rotation of the ring-shaped actuator R is controlled via the wiring g.

The coolant liquid circulation control unit 42 is to control operation of the coolant liquid circulating pump to circulate coolant liquid in accordance with data related to temperature control received by the Peltier device operation control unit 41. The coolant liquid circulation control unit 42 controls circulation of coolant liquid by the pump and the temperature of the coolant liquid in the warm liquid heat radiating part A_{w} via the wiring q. The coolant liquid circulation control unit 42 controls circulation of the coolant liquid by the pump and the temperature of the coolant liquid in the cold liquid heat radiating part A_{c} via the wiring p.

The cerebral local portion is efficiently cooled by radiating the heat generated due to the operation of the Peltier device 2 by the coolant liquid circulating through the coolant liquid circulating part 4. It is necessary that circulation of the coolant liquid is not stopped simultaneously after stopping the operation of the Peltier device but circulation of the coolant liquid is controlled to be continued for about 3 to 5 minutes. The reason for this is to inhibit a rapid rise of temperature of the probe caused by stopping the circulation of the coolant liquid simultaneously.

The temperature detection signals and the brain wave detection signals received by the Peltier device operation control unit 41 are transmitted to the data processing unit 43 in a form in which A/D conversion is performed and the data set and input by a setting-inputting board IB are transmitted to the data processing unit 43. The data processing unit 43 analyzes the data on the basis of the temperature detection signals and forms mapping data in accordance with the set and input data to accumulate and display the data on a display DP successively.

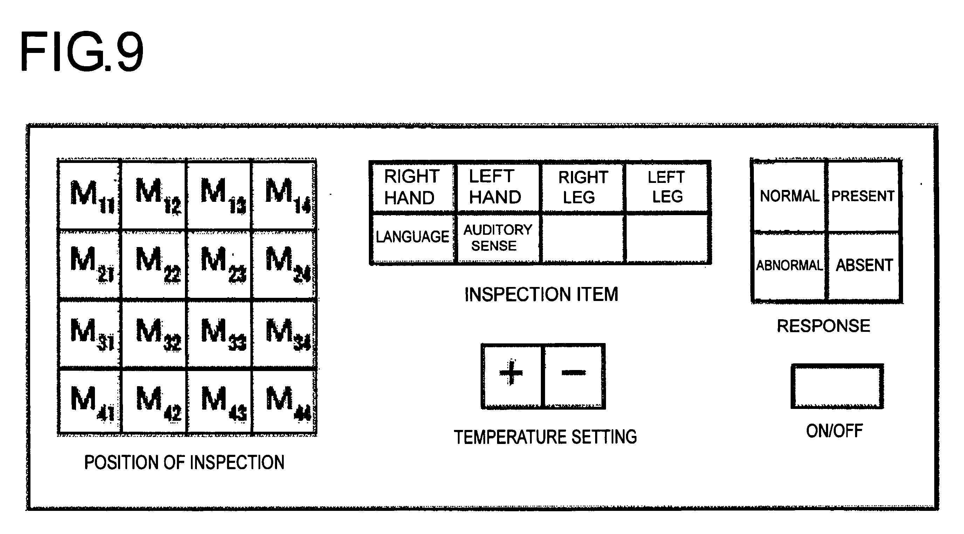

The setting-inputting board IB is used for selecting and inputting the inspection items or the like in mapping and configured to be a form as shown in FIG. 9, for example. In FIG. 9, in a left field of the inspection positions, buttons are arranged for specifying any of the plurality of cooling modules in the mapping module to be selected and used for inspection. The Peltier device of the cooling module selected by this button is switched by the switch SW to be operated via the wirings a, d and information showing what cooling module is selected are transmitted to the data processing unit 43.

As for the inspection items in the central field in GIG. 9, buttons are arranged for specifying the item to be inspected as a response to the cooling of a cerebral local portion by a selected cooling module. Also, in the right field, buttons are arranged for specifying and inputting contents of determination such as presence or absence of response and normal or abnormal in accordance with the responses at the cerebral local portions to be inspected for the respective inspection items. By selecting these buttons, results obtained by determining the responses for the respective inspection positions and the inspection items are input and transmitted to the data processing unit 43.

An arrangement of setting and inputting buttons in the setting and inputting board IB is in accordance with the contents such as inspection items by the cerebral function mapping device. The buttons for selecting cooling modules in the left field are in accordance with the arrangement and configuration of the cooling modules and the buttons for inspection items or responses are also arranged in accordance with their applied forms. The setting-inputting buttons may be in a form of a snap-type button or a touch-screen. In lower fields of the setting-inputting board, on/off switch for operation of the mapping device and a temperature setting part in cooling by the mapping module are arranged.

Functions of the setting-inputting board IB are realized in a form of hardware such as keys or buttons to be operated as well as in a form of software in which a program related to control of the cerebral function mapping device is formed so that a layout of predetermined operation items as the setting-inputting board is displayed on a display screen and the setting and inputting may be performed according to the key operations on a computer.

The operation procedure of the cerebral function mapping with the cerebral function mapping device configured as described above may be performed via similar steps in a case where the state of the cerebral local portion is monitored by the cerebral local portion cooling probe.
That is, the procedure is performed by steps of:
(a) Craniotomy is performed in a range necessary to map and excise lesion under general anesthesia.
(b) After completing the craniotomy, the anesthesia is discontinued to obtain an awakened state of a patient.
(c) Cerebral function is mapped with a focus on a language area or a motor area anatomically expected through making the tip end of the mapping module contact with the cerebral local portion and detecting brain wave signals with the cerebral local portion cooling probe set at a predetermined temperature in an operating state of the mapping module.
   In cooling, the temperature of the tip ends of the cooling modules of the mapping module are set at a predetermined temperature in a range of 5 to 15°C and changes in neurological symptoms (a language function and a motor function of limbs) of the patient are observed before, during and after the cooling.
(d) The language function is evaluated according to naming of objects. The motor function is evaluated according to elevation of an upper limb and a lower limb, enclasping and unclasping of a hand and toe movement.
(e) Brain waves of cooled sites and around the cooled sites are measured and changes in electrical activity are also observed.

In mapping, the cerebral function mapping device is launched by turning the on/off switch of the device in the setting and inputting board IB and the temperature of cooling by the mapping module is set by the temperature setting part. The temperature of the cooled cerebral local portion with respect to the set temperature is controlled by the operation of the Peltier device and the circulation of coolant liquid so that the result is displayed on the display. After confirming that temperature is the predetermined, the mapping is performed by using the mapping module.

First, one of the cooling modules M₁₁, M₁₂, ..., Mₘₙ is selected and specified by the button of the inspection position so that the Peltier device with respect to only the specified cooling module is in a cooling operation state and cold liquid is in a circulation state, while the Peltier devices of the other cooling modules are in a non-operation state and warm liquid is in a circulation state. In such states of the mapping module, the responses of a subject are successively examined. In this operation, when the cooling probe M₁₁ is specified, a response of a right hand is examined to push down a "right hand" button so as to input presence or absence of the response and normal or abnormal state of the right hand by pressing the buttons of response. In such operations, responses with respect to inspection items such as a left hand, a right foot or the like are successively examined and input by the buttons of response.

Data input as inspection items with the respective cooling modules and responses to the items are successively stored in a memory of the data processing unit so that the data of responses to the inspection items obtained with the whole cooling modules M₁₁, M₁₂, ..., Mₘₙ result in cerebral function mapping data.

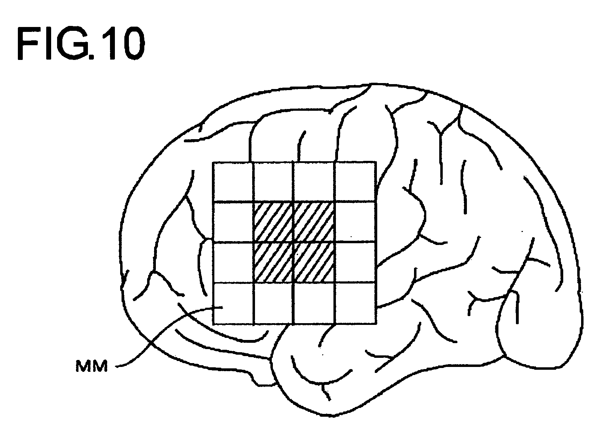

FIG. 10 shows an example of a case where the cerebral function mapping data are shown in which the part of the mapping module MM is displayed with an image of a brain combined therwith as a background on the display. In FIG. 10, a result is shown in which M₂₂, M₂₃, M₃₂, and M₃₃ of the mapping data related to the language area are abnormal.

The cerebral local portion cooling probe and the mapping device configured to include a plurality of the cooling modules according to the present invention are to operate the cooling means to perform cooling as stimulation to obtain a response with high safety and reliability without inducing convulsion fit as in a case where electrodes are used to cause stimulation.

### EXPLANATION OF REFERENCE NUMERALS

- 1: Casing
- 1a: Rear end wall
- 2: Peltier device
- 3: Metal plate
- 4: Coolant liquid circulating part
- 5a: Inflow conduit
- 5b: Outflow conduit
- 6: Temperature detecting element
- 7: Brain wave detecting electrode
- 8: Wiring
- 9: Wiring
- 10: Wiring
- 21: Coolant liquid circulating pump
- 22: Reservoir
- 23: Cooling part
- 31: Probe operation control unit
- 32: Coolant liquid circulation control unit
- 33: Switch setting and inputting part
- 34: Data processing unit
- 41: Peltier device operation controlling unit
- 42: Coolant liquid circulation controlling unit
- 43: Data processing unit
- M: Handheld module
- MM: Mapping module
- A: Heat radiating part
- A_{c}: Cold liquid heat radiating part
- A_{w}: Warm liquid heat radiating part
- B: Control unit
- CP: Coupling part
- CT: Selecting and coupling conduit
- CV: Flow path switch
- cw: Coupling conduit
- cc: Coupling conduit
- F: Casing
- R: Ring-shaped actuator
- RC: Joint
- RJ: Joint
- FJ: Joint
- IB: Setting and inputting board
- DP: Display

## Claims

1. A cerebral local portion cooling probe, comprising:
a handheld module having a tubular casing and cooling means and temperature detecting means which are provided in the tubular casing at a tip end thereof; and
a cooling control unit controlling temperature of a local portion of a cerebral surface to a predetermined temperature in accordance with temperature detection signal from the temperature detecting means, with the tip end of the handheld module being caused to contact with a cerebral local portion in order to cool the cerebral surface to the predetermined temperature thereby monitoring a state of the cerebral local portion,
wherein the cooling means provided in the tubular casing at the tip end thereof is a Peltier device,
a coolant liquid circulating part is provided so as to contact with the heat generating surface on the rear side of the Peltier device, and
the coolant liquid circulating part and a heat radiating part provided externally of the handheld module are coupled by conduits to circulate coolant liquid and radiate heat.

2. The cerebral local portion cooling probe according to claim 1, wherein
a metal plate is provided at the front surface of the Peltier device and exposed as a tip end surface of the tubular casing, and wherein
a thermocouple and a brain wave detecting electrode are embedded in the metal plate as the temperature detecting means.

3. The cerebral local portion cooling probe according to claim 2,
wherein the metal plate is provided at the front surface of the Peltier device and exposed as the tip end surface of the tubular casing, and
wherein the thermocouple is embedded in the metal plate as the temperature detecting means.

4. The cerebral local portion cooling probe according to claim 3,
wherein the metal plate is used as a brain wave detecting electrode.

5. A cerebral function mapping device, comprising:
a mapping module accommodating a plurality of cooling modules in a casing so that cooling tip end surfaces of the cooling modules are arranged in a same plane, each of these cooling modules including a tubular casing, cooling means and temperature detecting means arranged in the tubular casing at the tip end thereof;
a cooling control unit for controlling temperature of a local portion of a brain surface at a predetermined temperature according to temperature detection signals from the temperature detecting means in selected one of the cooling modules of the mapping module;
cooling module selecting means for selecting one of the plurality of cooling modules in the mapping module and causing only the selected one of the cooling modules to perform cooling operation;
response inputting means for inputting information on responses of the cerebral local portion monitored in accordance with the cooling operation of the selected cooling module; and
mapping data creating means for accumulating the information input by the response inputting means and creating mapping data.

6. The cerebral function mapping device according to claim 5,
wherein the cooling means of the respective cooling modules in the mapping module is a Peltier device,
a coolant liquid circulating part is arranged to contact with the heat generating surface at the rear side of the Peltier device,
an inflow conduit and an outflow conduit of coolant liquid connected to the coolant liquid circulating part in the plurality of cooling modules can be connected to any one of a cold liquid heat radiating part and a warm liquid heat radiating part, and
wherein cold liquid of the coolant liquid is circulated through the selected one of the cooling modules and warm liquid of the coolant liquid is circulated through the other cooling modules so as to radiate heat at the cold liquid heat radiating part and the warm liquid heat radiating part respectively.

7. The cerebral function mapping device according to claim 6, wherein
a metal plate is provided at the front surface of the Peltier device of each of the cooling modules in the mapping module and exposed as the tip end surface of the tubular casing of each of the cooling modules, and wherein
a thermocouple is embedded in the metal plate as the temperature detecting means.

8. The cerebral function mapping device according to claim 7, wherein the metal plate at the tip end surface of each of the cooling modules of the mapping module is used as a brain wave detecting electrode.
